(19) Europäisches Patentamt
European Patent Office
Office européen des brevets

(11) Veröffentlichungsnummer: **0 073 443**
**B1**

(12) # EUROPÄISCHE PATENTSCHRIFT

(45) Veröffentlichungstag der Patentschrift: **05.11.86**

(51) Int. Cl.⁴: **C 07 D 285/00**, A 01 N 43/72

(21) Anmeldenummer: **82107702.1**

(22) Anmeldetag: **23.08.82**

(54) **2H-1,2,4,6-Thiatriazin-1,1-dioxide, ein Verfahren zu ihrer Herstellung und ihre Verwendung zur Bekämpfung unerwünschten Pflanzenwuchses.**

(30) Priorität: **28.08.81 DE 3134143**

(43) Veröffentlichungstag der Anmeldung: **09.03.83 Patentblatt 83/10**

(45) Bekanntmachung des Hinweises auf die Patenterteilung: **05.11.86 Patentblatt 86/45**

(84) Benannte Vertragsstaaten: **AT BE CH DE FR GB IT LI NL**

(56) Entgegenhaltungen: **EP-A-0 029 908**

(73) Patentinhaber: **BASF Aktiengesellschaft, Carl-Bosch- Strasse 38, D-6700 Ludwigshafen (DE)**

(72) Erfinder: **Hamprecht, Gerhard, Dr., Rote- Turm-Strasse 28, D-6940 Weinheim (DE)**
Erfinder: **Wuerzer, Bruno, Dr. Dipl.- Landwirt, Ruedigerstrasse 13, D-6701 Otterstadt (DE)**

EP 0 073 443 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft 2H-1,2,4,6-Thiatriazin-1,1-dioxide, Verfahren zu ihrer Herstellung sowie Herbizide, die diese Verbindungen als Wirkstoffe enthalten.

Es ist bekannt, daß man substituierte 6H-1,2,4,6-Thiatria-zin(5)on-1,1-dioxidderivate erhält, wenn man beispielsweise Addukte aus 2 Mol Isocyanat und einem Mol Chlorsulfonylisocyanat mit Alkoholen umsetzt (DE-A- 1 946 262) oder wenn man N'-Carboalkoxy-N-sulfamoyl-guanidine oder -amidine unter alkalischen Bedingungen cyclisiert. Diese 6H-1,2,4,6-Thiatriazin(5)on-1,1-dioxidderivate sind herbizid wirksam (DE-A- 2 508 832, DE-A- 2 933 889).

Weiterhin sind in der EP-A- 29 908 herbizide Wirkstoffe beschrieben, die sich von 6H-1,2,4,6-Thiatriazin-1,1-dioxidderivaten ableiten, die in Ringposition 5 einen über ein Sauerstoff- oder Schwefelatom oder über eine Sulfinyl- oder Sulfonylgruppe gebundenen, gegebenenfalls substituierten Phenyl-, Benzyl- oder (cyclo)aliphatischen Rest aufweisen.

Es wurde gefunden, daß 2H-1,2,4,6-Thiatriazin-3-halogen-1,1-dioxide der Formel

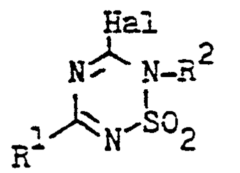

(I),

in der $R^1$ Wasserstoff, einen gesättigten oder ungesättigten unverzweigten aliphatischen Rest mit bis zu 10 Kohlenstoffatomen, einen gesättigten oder ungesättigten verzweigten aliphatischen Rest mit 3 bis 10 Kohlenstoffatomen, einen unverzweigten oder verzweigten, durch Halogen, Alkoxy oder Alkylmercapto mit 1 bis 4 Kohlenstoffatomen substituierten aliphatischen gesättigten Rest mit 2 bis 10 Kohlenstoffatomen, einen cycloaliphatischen Rest mit 3 bis 7 Kohlenstoffatomen, einen gegebenenfalls durch Halogen, Alkyl oder Alkoxy mit 1 bis 4 Kohlenstoffatomen substituierten Phenylrest oder einen gegebenenfalls halogensubstituierten Benzylrest, $R^2$ Wasserstoff, einen unverzweigten gesättigten oder ungesättigten aliphatischen Rest mit bis zu 10 Kohlenstoffatomen, einen verzweigten gesättigten oder ungesättigten aliphatischen Rest mit 3 bis 10 Kohlenstoffatomen, einen cycloaliphatischen Rest mit 3 bis 7 Kohlenstoffatomen, einen Halogenalkylrest mit 2 bis 10 Kohlenstoffatomen oder einen Alkoxyalkylrest mit 2 bis 6 Kohlenstoffatomen und Hal Halogen bedeuten,

eine selektive herbizide Wirkung haben und wertvolle Zwischenprodukte für die Herstellung von Farbstoffen, Pharmazeutika und von Wirkstoffen für Pflanzenschutzmittel sind.

$R^1$ in Formel I bedeutet Wasserstoff, einen gesättigten oder ungesättigten, unverzweigten aliphatischen Rest mit bis zu 10 Kohlenstoffatomen, vorzugsweise mit bis zu 4 Kohlenstoffatomen, oder einen gesättigten oder ungesättigten, verzweigten aliphatischen Rest mit 3 bis 10 Kohlenstoffatomen, vorzugsweise mit 3 oder 4 Kohlenstoffatomen, beispielsweise einen Alkyl-, einen Alkenyloder einen Alkinylrest mit bis zu 10 Kohlenstoffatomen, vorzugsweise mit bis zu 4 Kohlenstoffatomen, wie Methyl, Ethyl, n-Propyl, Isopropyl, n-Butyl, Isobutyl, sec.-Butyl, tert.-Butyl, n-Pentyl, n-Hexyl, 1-Ethyl-n-propyl, 1,2-Di-methyl-n-propyl, 1,3-Dimethyl-n-butyl, 1-Ethyl-2-methyl-n-propyl, 1,2,2-Trimethyl-n-propyl, 1,2-Dimethyl-n-hexyl, tert.-Amyl, Allyl, Methallyl, Crotyl, 2-Ethylhexen-2-yl, Hexen-5-yl, 2-Methyl-buten-2-yl, 2-Methyl-isobut-2-enyl, sec.-But-2-inyl, Butin-2-yl, Isobut-2-enyl, Propargyl, 2-Methyl-but-3-enyl, 2-Methyl=but-2-enyl, 1-Methyl-iso-but-2-enyl, einen cycloaliphatischen Rest, beispielsweise einen Cycloalkylrest mit 3 bis 7 Kohlenstoffatomen, wie Cyclopropyl, Cyclopentyl, Cyclohexyl, einen unverzweigten oder verzweigten,

durch Halogen, Alkoxy oder Alkylmercapto mit 1 bis 4 Kohlenstoffatomen substituierten gesättigten aliphatischen Rest mit 2 bis 10 Kohlenstoffatomen, vorzugsweise mit 2 bis 4 Kohlenstoffatomen, beispielsweise einen Halogenalkyl, Alkoxyalkyl- oder Alkylmercaptoalkylrest mit 2 bis 10 Kohlenstoffatomen, vorzugsweise mit 2 bis 4 Kohlenstoffatomen, wie 2-Chlorethyl, 2-Chlor-n-propyl, 3-Chlor-n-propyl, 2-Chlor-sec.-butyl, 2-Chlor-isopropyl, 2-Fluor-sec.-butyl, 2:Fluor-isobutyl, 2-Fluorisopropyl, 2-Chlor-tert.-butyl, 2,2,2-Trifluorethyl, Methoxyethyl, Ethoxyethyl, 3-Methoxy-n-propyl, Methoxy-isopropyl, 3-Methoxy-n-butyl, 1-Methoxy-ethyl-n-propyl, Methoxy-tert.-butyl, Ethoxy-tert.-butyl, 2-Methoxy-n-butyl, 4-Methoxy-n-butyl, Methylmercaptoethyl, Ethylmercapto-ethyl, 3-Methylmercapto-n-propyl, 3-Methylmercapto-n-butyl, 1-Methylmercapto-butyl-2, Methylmercapto-tert.-butyl, 2-Methylmercapto-butyl, einen gegebenenfalls durch Halogen, Alkyl oder Alkoxy mit 1 bis 4 Kohlenstoffatomen substituierten Phenylrest, wie Phenyl, 4-Chlorphenyl, 3,4-Dichlorphenyl, 4-tert.-Butylphenyl, 4-Methoxy-3-chlorphenyl, 4-Methoxyphenyl, 2-Methylphenyl, 2-Methyl-4-chlorphenyl, oder einen gegebenenfalls am Phenylring durch Halogen, wie Fluor, Chlor, Brom oder Iod, substituierten Benzylrest, wie Benzyl, 4-Chlorbenzyl, 2,6-Dichlorbenzyl, 2-Chlor-6-fluorbenzyl, 2,6-Difluor-benzyl. $R^2$ in Formel I bedeutet Wasserstoff, einen gesättigten oder ungesättigten, unverzweigten aliphatischen Rest mit bis zu 10 Kohlenstoffatomen, vorzugsweise mit bis zu 4 Kohlenstoffatomen, oder einen gesättigten oder ungesättigten verzweigten aliphatischen Rest mit 3 bis 10 Kohlenstoffatomen, vorzugsweise mit 3 oder 4 Kohlenstoffatomen, beispielsweise einen Alkyl-, einen Alkenyl- oder einen Alkinylrest mit bis zu iO Kohlenstoffatomen, vorzugsweise mit bis zu 4 Kohlenstoffatomen, wie Methyl, Ethyl, n-Propyl, Isopropyl, n-Butyl, Isobutyl, sec.-Butyl, tert.-Butyl, n-Pentyl, n-Hexyl, 1-Ethyl-n-propyl, 1,2-Di-methyl-n-propyl, 1,3-Dimethyl-n-butyl, 1-Ethyl-2-methyl-n-propyl, 1,2,2-Trimethyl-n-propyl, 1,2-Dimethyl-n-hexyl, tert.-Amyl, Allyl, Methallyl, Crotyl, 2-Ethylhexen-2-yl, Hexen-5-yl, 2-Methyl-buten-2-yl, 2-Methyl-isobut-2-enyl, sec.-But-2-inyl, Butin-2-yl, Isobut-2-enyl, Propargyl, 2-Methyl-but-3-enyl, 2-Methyl-but-2-enyl, i-Methyl-isobut-2-enyl, einen cycloaliphatischen Rest mit 3 bis 7 Kohlenstoffatomen, beispielsweise einen Cycloalkylrest mit 3 bis 7 Kohlenstoffatomen, wie Cyclopropyl, Cyclopentyl, Cyclohexyl, oder einen Halogenalkylrest mit 2 bis 10 Kohlenstoffatomen, vorzugsweise mit 2 bis 4 Kohlenstoffatomen, wie 2-Chlorethyl, 2-Chlor-n-propyl, 3-Chlor-n-propyl, 2-Chlor-sec.-butyl, 2-Chlor-isopropyl, 2-Fluor-sec.-butyl, 2-Fluor-isobutyl, 2-Fluorisopropyl, 2-Chlor-tert.-butyl, 2,2,2-Trifluorethyl, oder einen Alkoxyalkylrest mit 2 bis 6 Kohlenstoffatomen, vorzugsweise mit 2 bis 4 Kohlenstoffatomen, wie Methoxyethyl, Ethoxyethyl, 3-Methoxy-n-propyl, Methoxy-n-propyl, Methoxy-isopropyl, 3-Methoxy-n-butyl, 1-Methoxyethyl-n-propyl, Methoxy-tert.-butyl, Ethoxy-tert.-butyl, 2-Methoxy-n-butyl, 4-Methoxy-n-butyl.

Als Halogen für Hal in Formel I kommen Fluor, Chlor, Brom oder Iod in Betracht.

Bevorzugte Verbindungen der Formel I sind solche, bei denen $R^1$ und $R^2$ Alkylreste mit 1 bis 4 Kohlenstoffatomen, insbesondere Methyl oder Ethyl, und Hal Chlor bedeuten.

Man erhält die Verbindungen der Formel I durch Umsetzung von Verbindungen der Formel

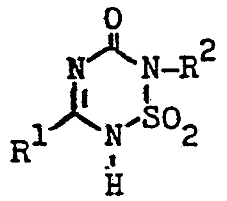

(II),

in der $R^1$ und $R^2$ die obengenannten Bedeutungen haben, oder deren Alkalimetallsalzen oder Erdalkalimetallsalzen mit einem Säurehalogenid der Phosphorsäure, phosphorigen Säure, Kohlensäure, Oxalsäure oder schwefligen Säure gegebenenfalls in Gegenwart eines Lösungs- oder Verdünnungsmittels und gegebenenfalls in Gegenwart eines Reaktionsbeschleunigers.

Verwendet man 2-Ethyl-5-methyl-2H-1,2,4,6-thiatriazin(3)-on-1,1-dioxid und Phosphorpentachlorid als Ausgangsmaterialien, so kann der Reaktionsablauf durch folgendes Formelschema wiedergegeben werden:

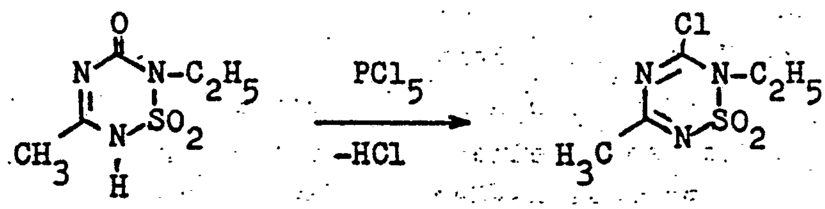


Zweckmäßigerweise verwendet man für die Umsetzung unter den Reaktionsbedingungen inerte Lösungs- oder Verdünnungsmittel, wie Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Tetrachlor-ethan, Trichlorethylen, Pentachlorethan, *o-, m-, p-Difluorbenzol, 1,2-Dichlorethan, 1,1-Dichlorethan, 1,2-cis-Dichlorethylen, Chlorbenzol, Fluorbenzol, Brombenzol, o-, m-, p-Dichlorbenzol, o-, m-, p-Dibrombenzol, o-, m-, p-Chlortoluol, 1,2,4-Trichlorbenzol, oder Ether, z.B. Ethylpropylether, Methyl-tert.-butylether, n-Butylether, Diisopropylether, Anisol, Dioxan, Ethylenglykoldimethylether, oder Nitro-kohlenwasserstoffe, z.B. Nitrobenzol, o-, m-, p-Chlornitrobenzol, o-Nitrotoluol, oder Nitrile, z.B. Acetonitril, Butyronitril, Isobutyronitril, Benzonitril, m-Chlorbenzonitril, oder aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Tri-methylpentan, 2,2,3-Trimethylpentan, oder Ester, z.B. Ethylacetat, und entsprechende Gemische. Geeignete Lösungsmittel sind ferner anorganische Säurechloride, z.B. Phosphoroxychlorid, oder entsprechende Mischungen mit inerten Chlorkohlenwasserstoffen, wie 1,2-Dichlorethan. Zweckmäßigerweise verwendet man das Lösungsmittel in einer Menge von 100 bis 2000 Gew.%, vorzugsweise von 200 bis 700 Gew.%, bezogen auf den Ausgangsstoff der Formel II.

Bevorzugte Säurehalogenide sind Thionylchlorid, Schwefeltetrafluorid, Phosgen, Oxalsäurechlorid, Phosphortribromid und insbesondere Phosphorpentachlorid, Phosphortrichlorid und Phosphoroxichlorid. Die Umsetzung wird im allgemeinen mit einer Menge von 1,0 bis 1,5, vorzugsweise 1,05 bis 1,2 Mol, Säurehalogenid, bezogen auf Ausgangsstoff II, durchgeführt; im Falle der Verwendung fünfwertiger

Phosphorhalogenverbindungen verwendet man 0,7 bis 1,5, vorzugsweise 1,0 bis 1,2 Mol, Phosphorpentahalogenid, bezogen auf Ausgangsstoff II.

Im Falle der Verwendung eines Phosphor(V)-halogenids als Halogenierungsagens empfiehlt sich die Verwendung eines Phosphoroxihalogenids als Verdünnungsmittel, das zweckmäßigerweise in einer Menge von 1 bis 10 Mol Phosphoroxihalogenid, bezogen auf Ausgangsstoff II, eingesetzt wird.

Hierbei kann das Phosphor(V)-halogenid auch direkt in situ hergestellt werden, indem man beispielsweise eine Mischung eines Phosphor(III)-halogenids in Phosphoroxihalogenid oder einem der obengenannten inerten Lösungsmittel mit der erforderlichen stöchiometrischen Menge aktivem Halogen umsetzt, beispielsweise nach dem in der US-PS 1 906 440 beschriebenen Verfahren, und dann nach Zugabe des Ausgangsstoffes II die Umsetzung durchführt.

Als Reaktionsbeschleuniger verwendet man vorteilhaft am Stickstoffatom disubstituiertes, gegebenenfalls cyclisches Carbonsäureamid, einen tetraalkylsubstituierten Harnstoff, ein tertiäres Amin, in einer Menge von 1 bis 10 Gew.%, bezogen auf den Ausgangsstoff II, verwenden. Auch Gemisch der genannten Katalysatoren kommen für die Reaktion in Betracht. Ferner kann man auch Salze von Diaminen, z.B. die Rydrochloride der Amine, oder ein quaternäres Salz eines Amins verwenden. Bevorzugte Katalysatoren sind:

Triethylamin, Pyridin, N,N-Dimethylanilin, M-Ethylpiperidin, M-Methylpyrrolidin, α, β- oder γ-Picolin, Chinolin, Isochinolin, Chinazolin, Chinoxalin, N-Propyldiisopropylamin, 2,6- und 2,4-Lutidin, N-(4-Pyridyl)-pyridiniumchlorid-hydrochlorid, p-Dimethylaminopyridin, Pyrimidin, Acridin, Dimethylformamid, Diethylformamid, Ameisensäure-N-methylanilid, N,N-Dimethylacetamid, N-Methylpyrrolidon, Tetramethylharnstoff.

Die als Ausgangsstoffe der Formel II benötigten 2H-1,2,4,6-Thiatriazin(3)on-1,1-dioxide sind teilweise bekannt oder lassen sich durch Umsetzung von N-Carbo-alkoxy-amidinen mit Aminosulfonylhalogeniden herstellen. Nach ihren spektroskopischen Daten liegen sie in der unter der Formel II wiedergegebenen Strukturform vor. Je nach Lösungsmittel können dabei jedoch auch gewisse Anteile der tautomeren Form

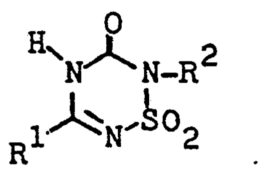

(IIa)

auftreten, die als im Gleichgewicht befindliche Verbindungen gleichermaßen bevorzugte Ausgangsmaterialien darstellen.

Zweckmäßigerweise wird das Verfahren zur Herstellung der Verbindungen der Formel I so durchgeführt, daß man den Ausgangsstoff II gegebenenfalls zusammen mit einem der vorgenannten inerten Verdünnungsmittel vorlegt, das Halogenierungsagens bei 0 bis 60°C, vorzugsweise bei 20 bis 40°C, zugibt und dann je nach Stärke der Gasentwicklung erhitzt.

Man kann jedoch auch den Ausgangsstoff II, gegebenenfalls in einem der obengenannten inerten Verdünnungsmittel, zu dem Halogenierungsagens zugeben. Im Falle des Phosgens empfiehlt sich die Zugabe eines Reaktionsbeschleunigers, beispielsweise von Dimethylformamid.

Zur Beendigung der Umsetzung rührt man noch 0,5 bis 18 Stunden bei 0 bis 160°C, vorzugsweise bei 60 bis 130°C, nach. Der Umsetzungsgrad läßt sich hierbei in einfacher Weise durch spektroskopische Methoden, beispielsweise anhand der Verschiebung der Protonenresonanzsignale der Reste $R^2$ oder $R^1$ verfolgen.

Aus dem Reaktionsgemisch wird der Endstoff I in üblicher Weise, z.B. nach Abdestillieren von Lösungsmittel und überschüssigem Halogenierungsagens, isoliert. Die gewünschten Endstoffe fallen hierbei in reiner Form an, gegebenenfalls können sie durch Umkristallisation, Chromatographie oder Destillation gereinigt werden. Wenn $R^1$ in der Formel II für eine basische Aminogruppe steht, fallen die Endprodukte je nach Aufarbeitung in der Regel in Form ihrer Hydrochloride an, aus denen man durch Waschen in basischem Medium freie Basen erhält. Die folgenden Vorschriften betreffen die Herstellung Verbindungen der Formel I.

**Beispiel 1**

3-Chlor-2,5-dimethyl-2H-1,2,4,6-thiatriazin-1,1-dioxid

212 Teile 2,5-Dimethyl-2H-1,2,4,6-thiatriazin(3)on-1,1-dioxid wurden bei Raumtemperatur in eine Mischung aus 292 Teilen Phosphorpentachlorid und 1400 Teilen Phosphoroxichlorid unter Rühren eingeführt und innerhalb 30 Minuten auf 110°C erhitzt. Nach 6 Stunden Rühren unter Rückfluß wurde das Reaktionsgemisch unter vermindertem Druck eingeengt, wobei 234 Teile (Ausbeute: 100 % d. Th.) 3-Chlor-2,5-dimethyl-2H-1,2,4,6-thiatriazin-1,1-dioxid vom Fp. 86 bis 90°C erhalten wurden (NMR (60 MHz, $CDCl_3$): $CH_3$-C 2,4δ, $CH_3$-N 3,68δ). (Verbindung Nr. 1).

**Beispiel 2**

3-Chlor-2-ethyl-5-methyl-2H-1,2,4,6-thiatriazin-1,1-dioxid

225 Teile 2-Ethyl-5-methyl-2H-1,2,4,6-thiatriazin(3)on-1,1-dioxid wurden innerhalb 2 Minuten unter Rühren in eine Mischung von 1300 Teilen Phosphoroxichlorid und 292 Teilen Phosphorpentachlorid gegeben. Das Reaktionsgemisch wurde 7 Stunden unter Rückfluß gerührt und dann unter vermindertem Druck eingeengt. Das verbleibende Öl wurde in 350 Teilen 1,2-Dichlorethan aufgenommen und über neutrales Aluminiumoxid (Aktivität I) chromatographiert. Nach dem Einengen wurden 230 Teile (Ausbeute: 93 % d. Th.) 3-Chlor-2-ethyl-5-methyl-2H-1,2,4,6-thiatriazin-1,1-dioxid vom Fp. 57 bis 59°C erhalten. (NMR (60 MHz, $CDCl_3$) $CH_3$-C 2,3 δ, N-$CH_2$ 3,68 - 4,03δ(q), $CH_3$-C 1,2 - 1,43δ(t) (Verbindung Nr. 2).

**Beispiel 3**

3-Chlor-2-methyl-5-ethyl-2H-1,2,4,6-thiatriazin-1,1-dioxid

110 Teile Phosphorpentachlorid wurden unter Rühren bei Raumtemperatur zu einer Mischung von 86 Teilen 2-Methyl-5-ethyl-2H-1,2,4,6-thiatriazin(3)on-1,1-dioxid in 100 Teilen 1,2-Dichlorethan und 112 Teilen Phosphoroxichlorid gegeben. Die Reaktionsmischung wurde 12 Stunden unter Rückfluß gerührt. Nach dem Einengen wurden 94 Teile (Ausbeute: 100 % d.Th.) 3-Chlor-2-methyl-5-ethyl-2H-1,2,4,6-thiatriazin-1,1-dioxid vom $n_D^{25}$ 1,5331 erhalten. NMR (60 MHz, $CDCl_3$): $CH_3$-N 3,7δ. Das Reinstprodukt siedet bei 123 bis 125°C/0,2 mbar mit $n_D^{25}$ 1,5337. (Verbindung Nr. 3).

Analog Beispiel 1 wurden die folgenden Verbindungen der Formel I erhalten:

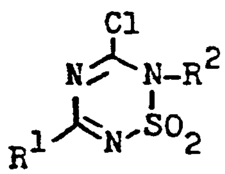

| Verbindung Nr. | $R^1$ | $R^2$ | physikalische Daten |
|---|---|---|---|
| 6 | $CH_3$ | i-$C_3H_7$ | $Kp_{0,2}$ = 123-127°C |
| 20 | $C_2H_5$ | $CH_2$-$CH_2Cl$ | $Kp_{0,2}$ = 133°C/$n_D^{25}$=1,5422 |
| 22 | n-$C_3H_7$ | $CH_3$ | $n_D^{25}$ = 1,5255 |
| 31 | i-$C_3H_7$ | $CH_3$ | $Kp_{0,2}$ = 116°C/Fp=62-67°C |
| 33 | i-$C_3H_7$ | n-$C_3H_7$ | $Kp_{0,2}$ = 123°C |
| 44 | i-$C_4H_9$ | $CH_3$ | $Kp_{0,2}$ = 119-120°C |
| 68 | Phenyl | $CH_3$ | $Kp_{0,2}$ = 178-182°C |
| 78 | $CH_3$ | n-$C_3H_7$ | $Kp_{0,3}$ = 109°C |

Analog Beispiel 1 können beispielsweise die folgenden Verbindungen der Formel I erhalten werden:

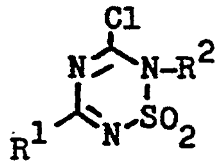

| Verbindung Nr. | $R^1$ | $R^2$ |
|---|---|---|
| 5 | H | $CH_3$ |
| 7 | $CH_3$ | sec-$C_4H_9$ |
| 8 | $CH_3$ | i-$C_4H_9$ |
| 9 | $CH_3$ | tert.-$C_4H_9$ |
| 10 | $CH_3$ | $C_6H_{11}$ |
| 11 | $CH_3$ | $CH_2$-$CH_2Cl$ |
| 12 | $CH_3$ | $CH_2$-$CH_2$-O-$CH_3$ |
| 13 | $C_2H_5$ | $C_2H_5$ |
| 14 | $C_2H_5$ | n-$C_3H_7$ |
| 15 | $C_2H_5$ | i-$C_3H_7$ |
| 16 | $C_2H_5$ | n-$C_4H_9$ |
| 17 | $C_2H_5$ | i-$C_4H_9$ |
| 18 | $C_2H_5$ | sec-$C_4H_9$ |
| 19 | $C_2H_5$ | tert.-$C_4H_9$ |
| 21 | $C_2H_5$ | $C_6H_{11}$ |
| 23 | n-$C_3H_7$ | $C_2H_5$ |
| 24 | H | H |
| 25 | n-$C_3H_7$ | n-$C_3H_7$ |
| 26 | n-$C_3H_7$ | i-$C_3H_7$ |
| 27 | n-$C_3H_7$ | sec-$C_4H_9$ |
| 28 | n-$C_3H_7$ | tert.-$C_4H_9$ |
| 29 | n-$C_3H_7$ | $CH_3$-O-$CH_2$-$CH_2$ |
| 30 | n-$C_3H_7$ | $C_6H_{11}$ |

| Verbindung Nr. | $R^1$ | $R^2$ |
|---|---|---|
| 32 | $i-C_3H_7$ | $C_2H_5$ |
| 34 | $i-C_3H_7$ | $i-C_3H_7$ |
| 35 | $i-C_3H_7$ | $sec.-C_4H_9$ |
| 36 | $i-C_3H_7$ | $C_6H_{11}$ |
| 37 | $n-C_4H_9$ | $CH_3$ |
| 38 | $n-C_4H_9$ | $C_2H_5$ |
| 39 | $n-C_4H_9$ | $n-C_3H_7$ |
| 40 | $n-C_4H_9$ | $i-C_3H_7$ |
| 41 | $n-C_4H_9$ | $sec.-C_4H_9$ |
| 42 | $n-C_4H_9$ | $ClCH_2-CH_2$ |
| 43 | $n-C_4H_9$ | $i-C_3H_7$ |
| 45 | $i-C_4H_9$ | $i-C_3H_7$ |
| 46 | $sec.-C_4H_9$ | $CH_3$ |
| 47 | $sec.-C_4H_9$ | $C_2H_5$ |
| 48 | $sec.-C_4H_9$ | $i-C_3H_7$ |
| 49 | $tert.-C_4H_9$ | $CH_3$ |
| 50 | $tert.-C_4H_9$ | $C_2H_5$ |
| 51 | $tert.-C_4H_9$ | $i-C_3H_7$ |
| 52 | $C_6H_{11}$ | $CH_3$ |
| 53 | $C_6H_{11}$ | $C_2H_5$ |
| 54 | $C_6H_{11}$ | $i-C_3H_7$ |
| 55 | $CH_2=CH-CH_2$ | $CH_3$ |
| 56 | $CH_2=CH-CH_2$ | $C_2H_5$ |
| 57 | $CH_2=CH-CH_2$ | $i-C_3H_7$ |
| 58 | Benzyl | $CH_3$ |
| 59 | Benzyl | $i-C_3H_7$ |
| 60 | 4-Chlor-benzyl | $CH_3$ |

| Verbindung Nr. | $R^1$ | $R^2$ |
|---|---|---|
| 61 | $CH_3$-O-$CH_2$-$CH_2$ | $CH_3$ |
| 62 | $CH_3$-O-$CH_2$-$CH_2$ | $C_2H_5$ |
| 63 | $CH_3$-S-$CH_2$-$CH_2$ | $CH_3$ |
| 64 | $CH_3$-S-$CH_2$-$CH_2$ | i-$C_3H_7$ |
| 65 | $CH_3$ | H |
| 66 | $C_2H_5$ | H |
| 67 | $CH_3$ | $CH_2$-CH=$CH_2$ |
| 69 | Benzyl | $CH_3$ |
| 70 | Phenyl | i-$C_3H_7$ |
| 71 | 4-Chlor-benzyl | $CH_3$ |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, asten, Stäubemitteln, Streumitteln oder Granulaten durch ersprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde,

Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung gemäß Beispiel 1 mit 10 Gewichtsteilen N-Methyl-α-pyrrolidon und erhält eine Lösung, die zur Anwendung in Form kleinster Tropfen geeignet ist.

II. 20-Gewichtsteile der Verbindung gemäß Beispiel 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung gemäß Beispiel 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 20 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthalt.

V. 20 Gewichtsteile der Verbindung Nr. 6 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 31 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitund des Wirkstoffs mit guter Haftfähigkeit.

VIII. 20 Teile der Verbindung Nr. 1 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder bei Nachauflaufanwendung erfolgen. Dabei können die Mittel auf den Standort aufgebracht werden, bevor die unerwünschten Pflanzen aus den Samen gekeimt oder aus vegetativen Pflanzenteilen ausgetrieben haben, oder sie werden auf die Blätter der unerwünschten Pflanzen und der Kulturpflanzen appliziert. Vorzugsweise werden die neuen Wirkstoffe nach dem Auflaufen oder während des Auflaufens der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,1 bis 15 kg/ha und mehr, vorzugsweise 0,5 bis 4,0 kg/ha, wobei sich die höheren Dosen besonders zur totalen Bekämpfung von Vegetationen eignen.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird. Zum Zwecke der Nachauflaufbehandlung zieht man die Testpflanzen je nach Wuchsform erst bis zu einer Wuchshöhe von 3 bis 10 cm an und behandelt sie danach.

Die Versuchsgefäße werden im Gewächshaus aufgestellt, wobei für wärmeliebende Arten wärmere Bereiche (20 bis 30°C) und für solche gemäßigter Klimate 15 bis 25°C bevorzugt werden. Die Versuchsperiode erstreckt sich über 3 bis 5 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 4 keine Schädigung oder normaler Auflauf und 100 kein Aufgang der Pflanzen bzw. völlige Zerstörung zumindest

der oberirdischen Teile.

Bei den Topfpflanzen handelt es sich um Chenopodium album (Weißer Gänsefuß), Datura stramonium (gemeiner Stechapfel), Galium aparine (Klettenlabkraut), Matricaria spp. (Kamille-Arten), Nicandra physaloides (Giftbeere), Oryza sativa (Reis), Sinapis alba (Weißer Senf), Solanum nigrum (schwarzer Nachtschatten), Zea mays (Mais), Gossypium hirsutum (Baumwolle).

Die Ergebnisse dieser Gewächshausversuche zeigen, daß die Verbindung Nr. 1 bei Vorauflaufanwendung von 2,0 kg Wirkstoff/ha eine gute herbizide Wirkung zeigt. Gleichzeitig werden einige Kulturpflanzen in selektiver Weise weitgehend geschont.

In Anbetracht der guten Verträglichkeit der Wirkstoffe und der Vielseitigkeit der Applikationsmethoden können die erfindungsgemäßen Verbindungen oder diese enthaltende Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |

| Botanischer Name | Deutscher Name |
|---|---|
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |

| Botanischer Name | Deutscher Name |
|---|---|
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |

| Botanischer Name | Deutscher Name |
|---|---|
| Vicia fabá | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmändeln eingesetzt werden. Zur Aktivierung der herbiziden Wirkung können auch Netz- und Haftmittel sowie nicht-phytotoxische Öle und Ölkonzentrate zugesetzt werden.

**Patentansprüche**

1. 2H-1,2,4,6-Thiatriazin-1,1-dioxide der Formel

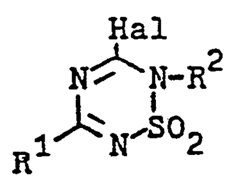

(I),

in der

$R^1$ Wasserstoff, einen gesättigten oder ungesättigten unverzweigten aliphatischen Rest mit bis zu 10 Kohlenstoffatomen, einen gesättigten oder ungesättigten verzweigten aliphatischen Rest mit 3 bis 10 Kohlenstoffatomen, einen unverzweigten oder verzweigten, durch Halogen, Alkoxy oder Alkylmercapto mit 1 bis 4 Kohlenstoffatomen substituierten aliphatischen gesättigten Rest mit 2 bis 10 Kohlenstoffatomen, einen cycloaliphatischen Rest mit 3 bis 7 Kohlenstoffatomen einen gegebenenfalls durch Halogen, Alkyl oder Alkoxy mit 1 bis 4 Kohlenstoffatomen substituierten Phenylrest oder einen gegebenenfalls halogensubstituierten Benzylrest,

$R^2$ Wasserstoff, einen unverzweigten aliphatischen, gesättigten oder ungesättigten Rest mit bis zu 10 Kohlenstoffatomen, einen verzweigten gesättigten oder ungesättigten aliphatischen Rest mit 3 bis 10 Kohlenstoffatomen, einen cycloaliphatischen Rest mit 3 bis 7 Kohlenstoffatomen, einen Halogenalkylrest mit 2 bis 10 Kohlenstoffatomen oder einen Alkoxyalkylrest mit 2 bis 6 Kohlenstoffatomen und Hal Halogen bedeuten.

2. 2H-1,2,4,6-Thiatriazin-1,1-dioxide der Formel I gemäß Anspruch 1, dadurch gekennzeichnet, daß $R^1$ und $R^2$ für Alkyl mit 1 bis 4 Kohlenstoffatomen oder Wasserstoff und Hal für Chlor stehen.

3. 3-Chlor-2,5-dimethyl-2H-1,2,4,6-thiatriazin-1,1-dioxid.

4. Herbizid, enthaltend ein 2H-1,2,4,6-Thiatriazin-1,1-dioxid der Formel I gemäß Anspruch 1 als Wirkstoff.

5. Herbizid, enthaltend inerte Zusatzstoffe und ein 2H-1,2,4,6-Thiatriazin-1,1-dioxid der Formel

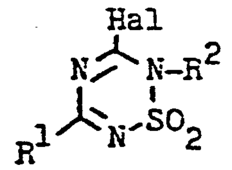 (I),

in der

$R^1$ Wasserstoff, einen gesättigten oder ungesättigten unverzweigten aliphatischen Rest mit bis zu 10 Kohlenstoffatomen, einen gesättigten oder ungesättigten verzweigten aliphatischen Rest mit 3 bis 10 Kohlenstoffatomen, einen unverzweigten oder verzweigten, durch Halogen, Alkoxy oder Alkymercapto mit 1 bis 4 Kohlenstoffatomen substituierten aliphatischen gesättigten Rest mit 2 bis 10 Kohlenstoffatomen, einen cycloaliphatischen Rest mit 3 bis 7 Kohlenstoffatomen, einen gegebenenfalls durch Halogen, Alkyl oder Alkoxy mit 1 bis 4 Kohlenstoffatomen substituierten Phenylrest oder einen gegebenenfalls halogensubstituierten Benzylrest,

$R^2$ Wasserstoff, einen unverzweigten aliphatischen, gesättigten oder ungesättigten Rest mit bis zu 10 Kohlenstoffatomen, einen verzweigten gesättigten oder ungesättigten aliphatischen Rest mit 3 bis 10 Kohlenstoffatomen, einen cycloaliphatischen Rest mit 3 bis 7 Kohlenstoffatomen, einen Halogenalkylrest mit 2 bis 10 Kohlenstoffatomen oder einen Alkoxyalkylrest mit 2 bis 6 Kohlenstoffatomen und Hal Halogen bedeuten,

als Wirkstoff.

6. Verfahren zur Bekämpfung unerwünschten Pflanzenwuchses, dadurch gekennzeichnet, daß man die Pflanzen und/oder ihren Standort mit einer herbizid wirksamen Menge eines 2H-1,2,4,6-Thiatriazin-1,1-dioxid der Formel I gemäß Anspruch 1 behandelt.

7 Verfahren zur Herstellung von 2H-1,2,4,6-Thiatriazin-1,1-dioxiden der Formel I gemäß Anspruch 1, dadurch gekennzeichnet, daß man Verbindungen der Formel II

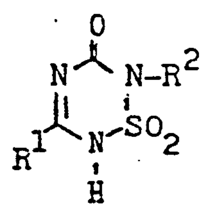 (II),

in der

$R^1$ und $R^2$ die im Anspruch 1 genannten Bedeutungen haben,

oder deren Alkalimetallsalze oder Erdalkalimetallsalze mit einem Säurehalogenid der Phosphorsäure, phosphorigen Säure, Kohlensäure, Oxalsäure oder schwefligen Säure gegebenenfalls in Gegenwart eines Lösungs- oder Verdünnungsmittels und gegebenenfalls in Gegenwart eines Reaktionsbeschleunigers ausgewählt aus der Gruppe, bestehend aus am Stickstoffatom disubstituierten Carbonsäureamiden tetraalkylsubstituierten Harnstoffen, tertiären Aminen, Salzen von Diaminen und quaternären Salzen von Aminen, umsetzt.

8. Verfahren nach Anspruch 7, dadurch gekennzeichnet, daß man die Umsetzung mit Phosphorpentahalogenid in einer Menge von 0,7 bis 1,5 Mol, bezogen auf 1 Mol Ausgangsstoff II, in Gegenwart von 1 bis 10 Mol Phosphoroxihalogenid, bezogen auf Ausgangsstoff II, als Lösungsmittel durchführt.

**Claims**

1. A 2H-1,2,4,6-thiatriazine-1,1-dioxide of the formula

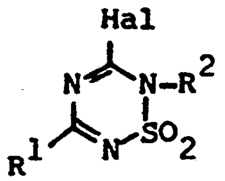

(I),

where

$R^1$ is hydrogen, a saturated or unsaturated straight-chain aliphatic radical of up to 10 carbon atoms, a saturated or unsaturated branched aliphatic radical of 3 to 10 carbon atoms, a straight-chain or branched saturated aliphatic radical of 2 to 10 carbon atoms which is substituted by halogen, alkoxy or alkylmercapto of 1 to 4 carbon atoms, a cycloaliphatic radical of 3 to 7 carbon atoms, phenyl which is unsubstituted or substituted by halogen, alkyl or alkoxy of 1 to 4 carbon atoms, or unsubstituted or halogen-substituted benzyl, $R^2$ is hydrogen, a straight-chain saturated or unsaturated aliphatic radical of up to 10 carbon atoms, a branched saturated or unsaturated aliphatic radical of 3 to 10 carbon atoms, a cycloaliphatic radical of 3 to 7 carbon atoms, a haloalkyl radical of 2 to 10 carbon atoms or an alkoxyalkyl radical of 2 to 6 carbon atoms, and Hal is halogen.

2. A 2H-1,2,4,6-thiatriazine-1,1-dioxide of the formula I as claimed in claim 1, where $R^1$ and $R^2$ are each alkyl of 1 to 4 carbon atoms or hydrogen, and Hal is chlorine.

3. 3-Chloro-2,5-dimethyl-2H-1,2,4,6-thiatriazine-1,1-dioxide.

4. A herbicide containing as active ingredient, a 2H-1,2,4,6-thiatriazine-1,1-dioxide of the formula I as claimed in claim 1.

5. A herbicide containing inert additives and, as active ingredient, a 2H-1,2,4,6-thiatriazine-1,1-dioxide of the formula

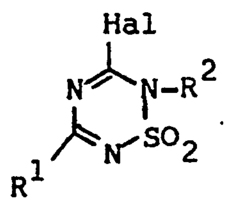

(I),

where

$R^1$ is hydrogen, a saturated or unsaturated straight-chain aliphatic radical of up to 10 carbon atoms, a saturated or unsaturated branched aliphatic radical of 3 to 10 carbon atoms, a straight-chain or branched saturated aliphatic radical of 2 to 10 carbon atoms which is substituted by halogen, alkoxy or alkylmercapto of 1 to 4 carbon atoms, a cycloaliphatic radical of 3 to 7 carbon atoms, phenyl which is unsubstituted or substituted by halogen, alkyl or alkoxy of 1 to 4 carbon atoms, or unsubstituted or halogen-substituted benzyl, $R^2$ is hydrogen, a straight-chain saturated or unsaturated aliphatic radical of up to 10 carbon atoms, a branched saturated or unsaturated aliphatic radical of 3 to 10 carbon atoms, a cycloaliphatic radical of 3 to 7 carbon atoms, a haloalkyl radical of 2 to 10 carbon atoms or an alkoxyalkyl radical of 2 to 6 carbon atoms, and Hal is halogen.

6. A process for combating the growth of unwanted plants, wherein the plants and/or their location are treated with a herbicidally effective amount of a 2H-1,2,4,6-thiatriazine-1,1-dioxide of the formula I as claimed in claim 1.

7. A process for the preparation of a 2H-1,2,4,6-thiatria-zine-1,1-dioxide of the formula I as claimed in claim 1, wherein a compound of the formula II

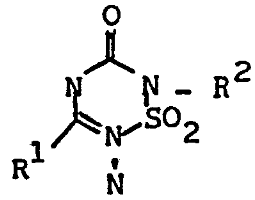

(II),

where

$R^1$ and $R^2$ have the meanings given in claim 1, or an alkali metal salt or alkaline earth metal salt thereof, is reacted with an acid halide of phosphoric acid, phosphorous acid, carbonic acid, oxalic acid or sulfurous acid,

in the presence or absence of a solvent or diluent, and in the presence or absence of a reaction accelerator selected from the group consisting of N-disubstituted carboxylic acid amides, tetraalkyl-substituted ureas, tertiary amines, salts of diamines and quaternary salts of amines.

8. A process as claimed in claim 7, wherein the reaction is carried out with a phosphorus(V) halide in an amount of from 0.7 to 1.5 moles per mole of starting material II, in the presence of 1 to 10 moles of phosphorus oxyhalide per mole of starting material II, as solvent.

**Revendications**

1. 1, 1-Dioxydes de la 2H-1,2,4,6-thiatriazine de la formule

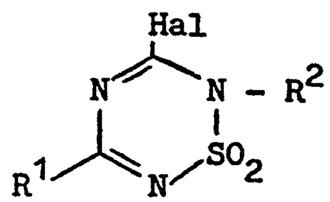

(I),

dans laquelle

$R^1$ désigne un atome d'hydrogène, un groupe aliphatique non ramifié, saturé ou non saturé, comportant jusqu'à dix atomes de carbone, un groupe aliphatique ramifié en $C_3$ à $C_{10}$, saturé ou non saturé, un groupe aliphatique saturé en $C_2$ à $C_{10}$, ramifié ou non, substitué par des substituants halogène, alcoxy ou alkylmercapto en $C_1$ à $C_4$, un groupe cycloaliphatique en $C_3$ à $C_7$, un groupe phényle éventuellement halo-, alkyl- ou alcoxy(en $C_1$ à $C_4$)-substitué ou un groupe benzyle éventuellement halo-substitué,

$R^2$ désigne un atome d' hydrogène ou un groupe aliphatique non ramifié, saturé ou non saturé, comportant jusqu'à dix atomes de carbone, un reste aliphatique ramifié en $C_3$ à $C_{10}$, saturé ou non saturé, un groupe cycloaliphatique en $C_3$ à $C_7$, un radical halo-alkyle en $C_2$ à $C_{10}$ ou un radical alcoxy-alkyle en $C_2$ à $C_6$ et Hal désigne un atome d'halogène.

2. 1,1-Dioxydes de la 2H-1,2,4,6-thiatriazine de la formule I selon la revendication 1, caractérisés en ce que $R^1$ et $R^2$ représentent un radical alkyle en $C_1$ à $C_4$ ou un atome d'hydrogène et Hal = Cl.

3. 1,1-Dioxyde de la 3-chloro-2,5-diméthyl-2H-1,2,4,6-thiatriazine.

4. Composition herbicide, contenant comme principe actif un 1,1-dioxyde de la 2H-1,2,4,6-thiatriazine de la formule I selon la revendication 1.

5. Composition herbicide, contenant comme principe actif un 1,1-dioxyde de la 2H-1,2,4,6-thiatriazine de la formule

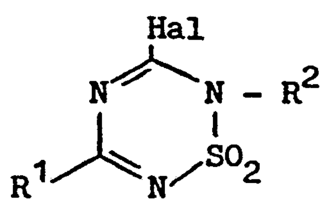

(I),

dans laquelle

$R^1$ désigne un atome d'hydrogène, un groupe aliphatique non ramifié, saturé ou non saturé, comportant jusqu'à dix atomes de carbone, un groupe aliphatique ramifié en $C_3$ à $C_{10}$, saturé ou non saturé, un groupe aliphatique saturé en $C_2$ à $C_{10}$, ramifié ou non, substitué par des substituants halogène, alcoxy ou alkylmercapto en $C_1$ à $C_4$, un groupe cycloaliphatique en $C_3$ à $C_7$, un groupe phényle éventuellement halo-, alkyl- ou alcoxy(en $C_1$ à $C_4$)-substitué ou un groupe benzyle éventuellement halo-substitué,

$R^2$ désigne un atome d'hydrogène ou un groupe aliphatique non ramifié, saturé ou non saturé, comportant jusqu'à dix atomes de carbone, un reste aliphatique ramifié en $C_3$ à $C_{10}$, saturé ou non saturé, un groupe cycloaliphatique en $C_3$ à $C_7$, un radical halo-alkyle en $C_2$ à $C_{10}$ ou un radical alcoxy-alkyle en $C_2$ à $C_6$ et Hal désigne un atome d'halogène, ainsi que des additifs inertes.

6. Procédé pour combattre le développement de plantes indésirables, caractérisé en ce que l'on traite les plantes et (ou) leur biotope avec une quantité efficace du point de vue herbicide d'un 1,1-dioxyde de la 2H-1,2,4,6-thiatriazine de la formule I selon la revendication 1.

7. Procédé de préparation de 1,1-dioxydes de la 2H-1,2,4,6-thiatriazine de la formule I selon la revendication 1, caractérisé en ce que l'on fait réagir des composés de la formule II

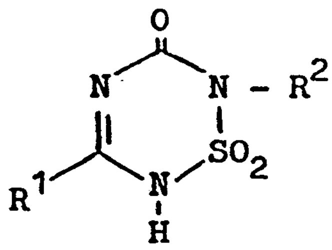

(II),

dans laquelle $R^1$ et $R^2$ possèdent les significations définies dans la revendication 1, ou des sels de métaux alcalins ou alcalino-terreux de tels composes, le cas échéant en présence d'un solvant ou d'un diluant et éventuellement en présence d'un accélérateur de la réaction, choisi parmi les amides carboxyliques disubstitués sur l'atome d' azote, les urées tétraalkyl-substituées, les amines tertiaires, les sels de diamines et les sels quaternaires d'amines,

avec un halogénure d'acide de l'acide phosphorique, de l'acide phosphoreux, de l'acide carbonique, de l'acide oxalique ou de l'acide sulfureux.

8. Procédé suivant la revendication 7, caractérisé en ce que la réaction est effectuée avec 0,7 mole à 1,5 mole par mole de composé de départ de la formule II de pentahalogénure de phosphore en présence de 1 à 10 moles par rapport au composé de départ II d'oxyhalogénure de phosphore comme solvant.